# EUROPÄISCHE PATENTANMELDUNG

(11) **EP 4 353 162 A1**
(43) Veröffentlichungstag der Anmeldung: **17.04.2024**
(21) Anmeldenummer: 23203395.1
(22) Anmeldetag: 13.10.2023
(51) Int. Cl.: A61B 17/22, A61B 17/00

(54) **MEDIZINISCHES INSTRUMENT ZUM BEHANDELN EINES KÖRPERS MIT EINER DRUCKENTLASTUNGSEINRICHTUNG**

(30) Priorität: 14.10.2022 DE 102022126989
(71) Anmelder: Karl Storz SE & Co. KG, 78532 Tuttlingen (DE)
(72) Erfinder: KRATTIGER, Beat, 78532 Tuttlingen (DE); LEHMANN, Simon, 78532 Tuttlingen (DE)

(57) **Zusammenfassung**

Die Erfindung betrifft ein medizinisches Instrument zum Behandeln eines Körpers, wobei das medizinische Instrument eine Trägereinheit mit einer Außenseite zu einer Umgebung und mindestens einen ersten Innenraum innerhalb der Trägereinheit aufweist, und der Innenraum mit einem Überdruck beaufschlagbar ist, wobei das medizinische Instrument eine Druckentlastungseinrichtung mit mindestens einem elastischen Dichtelement aufweist, wobei das mindestens eine elastische Dichtelement in einer ersten Aussparung in und/oder an der Außenseite der Trägereinheit angeordnet ist und der mindestens eine Innenraum mittels zumindest einem Druckentlastungskanal mit der ersten Aussparung verbunden ist, sodass bei einem Beaufschlagen des mindestens einen Innenraums mit dem Überdruck der Überdruck mittels eines Freigebens der ersten Aussparung durch das mindestens eine elastische Dichtelement in die Umgebung abgebbar ist.

## Beschreibung

Die Erfindung betrifft ein medizinisches Instrument zum Behandeln eines Körpers, wobei das medizinische Instrument eine Trägereinheit mit einer Außenseite zu einer Umgebung und mindestens einen ersten Innenraum innerhalb der Trägereinheit aufweist, und der Innenraum mit einem Überdruck beaufschlagbar ist.

Bei medizinischen Instrumenten wird häufig ein Druckmedium in einem Innenraum des Instrumentes eingesetzt, wobei ein hohles Werkzeug oder eine Hohlsonde zumindest teilweise in den Patienten eingeführt wird. Bei einem ungewollten Austritt von Druckmedium, wie beispielsweise von Druckluft oder einem Wärmefluid, aus dem Innenraum des Instrumentes in das hohle Werkzeug oder die Hohlsonde besteht ein potentielles Risiko für den Patienten. Bei einem Instrumentenfehler kann unbeabsichtigt beispielsweise Druckluft in den Körper des Patienten gelangen und dadurch den Patienten mit Keimen infizieren und/oder durch den Überdruck mechanisch verletzen.

Beispielsweise ist die Lithotripsie ein bekanntes Verfahren zum Zertrümmern von Körpersteinen, welche sich z.B. durch Kondensation und/oder Auskristallisation von Salzen und Eiweißen als sogenannte Konkremente in Körperorganen, wie beispielsweise in der Blase oder Niere, bilden. Wenn diese Körpersteine zu groß für einen natürlichen Abgang sind und Beschwerden verursachen, müssen diese mit einem Lithotripter zerkleinert werden, sodass die zerkleinerten Steine durch natürliche Ausscheidung und/oder mittels einer Saug-Spül-Pumpe entfernt werden können. Bei ballistischen Lithotriptern wird ein Projektil innerhalb eines Beschleunigungsrohres beschleunigt und die kinetische Energie des Projektils über einen elastischen Stoß auf das proximale Ende einer Sonde und weiter auf deren distales Ende zum Fragmentieren eines Körpersteins übertragen. Unabhängig von der Art der Erzeugung der Bewegungsenergie findet bei ballistischen Lithotriptern durch die Bewegung des Projektils im Beschleunigungsrohr stets eine Kompression eines Gas- und/oder Luftvolumens statt und ein entsprechender Überdruck tritt auf, welcher bei einem Fehler im druckbeaufschlagten Bereich des Lithotripters bis zu einem Patienten durchschlagen kann. Das Patientenrisiko ist weiter erhöht bei pneumatischen Lithotriptern, bei denen gezielt Druckluft zur Beschleunigung des Projektils innerhalb des Beschleunigungsrohres eingesetzt wird. Gerade bei pneumatischen Lithotriptern besteht eine erhöhte Gefahr, dass Druckluft beispielsweise durch einen Fehler, wie durch bei der Aufbereitung falsch montierte Teile oder eine vergessene Dichtung, und/oder durch einen funktionalen Fehler, wie durch einen Ermüdungsbruch, Druckluft in den Körper des Patienten gelangen kann.

Aus der DE 195 00 893 A1 ist eine Vorrichtung zur Zertrümmerung von Konkrementen mit einer Hohlsonde bekannt, wobei die Hohlsonde aus einem Rohr, einem Sondenfuß und einer Hohlsondenspitze besteht, und diese drei Bauelemente ineinander steckbar und über eine Lötverbindung zueinander festgelegt sind. Der Sondenfuß weist distal- und proximalseitig au-ßen Scheibenpaare auf, zwischen denen ein als O-Ring ausgebildetes Dichtungselement angeordnet ist. Da sich der Sondenfuß im Betrieb in distaler Richtung bewegt, besteht prinzipiell die Gefahr, dass bei Versagen der Dichtungen ein Überdruck von einer pneumatischen Antriebsanordnung in den Hohlraum der Sonde und dadurch in den Patienten gelangt.

Vor allem bei ballistischen und/oder pneumatischen Lithotriptern mit einer Hohlsonde besteht der Nachteil, dass durch Anwenderfehler, wie einen falschen Zusammenbau der Teile und/oder vergessene Teile nach der Aufbereitung, oder ein Versagen eines Bauteils Druckluft in den Patienten gelangt und der Patient ungewollt über die Hohlsonde mit Druckluft beaufschlagt wird. Neben dem aktiven Einsatz von Druckluft zum Bewegen des Projektils und einem durch die Bewegung des Projektils vorliegenden komprimierten Luftvolumens kann ein Überdruck mit einer potentiellen Gefahr für den Patienten auch von einem Druckluftreservoir oder einer Federeinrichtung zur Repulsation des Projektils ausgehen. Im Falle eines distalseitigen Druckluftreservoirs ist dieses über eine Verbindung und ein Schaltventil mit dem Inneren des Beschleunigungsrohres verbunden, um nach einem distalseitigen Anschlag des Projektils das Projektil wieder zum proximalseitigen Anschlag zurückzubewegen. Aufgrund der zeitlich getakteten Druckluftstöße und des Schlaghammer-Prinzips besteht hierbei eine erhöhte Gefahr der Ermüdung von Bauteilen und des Auftretens von Undichtigkeiten.

Aufgabe der Erfindung ist es, den Stand der Technik zu verbessern.

Gelöst wird die Aufgabe durch ein medizinisches Instrument zum Behandeln eines Körpers, wobei das medizinische Instrument eine Trägereinheit mit einer Außenseite zu einer Umgebung und mindestens einen ersten Innenraum innerhalb der Trägereinheit aufweist, und der Innenraum mit einem Überdruck beaufschlagbar ist, wobei das medizinische Instrument eine Druckentlastungseinrichtung mit mindestens einem elastischen Dichtelement aufweist, wobei das mindestens eine elastische Dichtelement in einer ersten Aussparung in und/oder an der Außenseite der Trägereinheit angeordnet ist und der mindestens eine Innenraum mittels zumindest einem Druckentlastungskanal mit der ersten Aussparung verbunden ist, sodass bei einem Beaufschlagen des mindestens einen Innenraums mit dem Überdruck der Überdruck mittels eines Freigebens der ersten Aussparung durch das mindestens eine elastische Dichtelement in die Umgebung abgebbar ist.

Somit wird ein fehlertolerantes medizinisches Instrument mit einer Druckentlastungseinrichtung als Sicherheitseinrichtung bereitgestellt, bei dem im Falle eines Überdrucks dieser gezielt in die Umgebung abgebbar ist, wodurch eine Druckbeaufschlagung eines Patienten verhindert ist. Mithin wird mittels der Druckentlastungseinrichtung im Falle eines zu hohen Überdruckes in dem Innenraum durch gezielte Ableitung des Überdruckes mittels der Druckentlastungeinrichtung in die Umgebung ein Durchschlagen des Überdruckes bis in einen Patienten und folglich eine mechanische Schädigung und/oder eine Kontamination des Körpers des Patienten verhindert. Dadurch, dass das elastische Dichtelement in einer ersten Aussparung in und/oder an der Außenseite der Trägereinheit und somit eines Gehäuses angeordnet und diese Aussparung mittels eines Druckentlastungskanals mit dem Innenraum verbunden ist, wirkt das elastische Dichtelement sowohl als Überdruckventil als auch als Rückschlagventil und verhindert gleichzeitig ein Eindringen von Fluiden und/oder Verunreinigung von der Umgebung in den Druckentlastungskanal und den Innenraum. Im Falle eines zu hohen Überdruckes löst sich das elastische Dichtelement von der Aussparung und gibt diese zum Durchströmen und zur Entlüftung frei, wodurch der mindestens eine über den Druckentlastungskanal verbundene Innenraum druckentlastet wird.

Ein wesentlicher Gedanke der Erfindung beruht darauf, mindestens einen Innenraum in der Trägereinheit eines medizinischen Instrumentes, welcher mit dem Patienten in fluidkommunizierender Verbindung steht oder bei dem eine fluidkommunizierende Verbindung im Fehlerfall herstellbar ist, über eine Druckentlastungseinrichtung mit einem Druckentlastungskanal zu verbinden, welcher außen an und/oder in der Außenseite der Trägereinheit in einer Aussparung endet, in der mindestens ein elastisches Dichtelement angeordnet ist, sodass im Normaldruckbetrieb das elastische Dichtelement die Trägereinheit und somit die Aussparung, den verbundenen Druckentlastungskanal und den Innenraum von außen abdichtet, jedoch bei einem zu hohen Überdruck in dem Innenraum das elastische Dichtelement sich von seinem Dichtsitz an und/oder in der Aussparung löst und dadurch die Aussparung freigibt und somit der Überdrück aus dem Innenraum über den Druckentlastungskanal und die freigegebene Aussparung in die Umgebung um die Trägereinheit entweichen kann. Dadurch wird eine Sicherheitseinrichtung mittels der Druckentlastungseinrichtung realisiert, mit welcher ein Durschlagen eines Überdruckes bis in den Körper des Patienten bei Verwendung des medizinischen Instrumentes verhindert ist. Der die Entlüftung auslösende Überdruck überwindet insbesondere das Gewicht, die Eigenspannung des elastischen Dichtelementes und/oder optional je nach Anordnung des Dichtelementes an und/oder in der Aussparung die Haftreibungskräfte zwischen dem Dichtelement und einer Oberfläche der Aussparung.

Es ist besonders vorteilhaft, dass die Druckentlastungseinrichtung bei einem Überdruck erzeugt durch jegliche Medien und/oder Fluide verwendbar ist und somit ein Überdruck eines Gases, von Druckluft, von Wasser, eines Hydraulikfluides und/oder eines Wärmetransportmittels vermindert und der entsprechende Innenraum druckentlastet werden kann.

Prinzipiell ist herauszustellen, dass die Aufgabe auch durch die Trägereinheit alleine gelöst wird, ohne dass ein vollständiges medizinisches Instrument ausgebildet ist. Dieser Fall liegt insbesondere vor, wenn beispielsweise bei einem Lithotripter die Hohlsonde austauschbar und somit von der Trägereinheit lösbar ist. Ebenso kann ein Schaft und/oder ein Werkzeug des medizinischen Instrumentes von der Trägereinheit lösbar und somit austauschbar sein, sodass die Trägereinheit auch alleine vorliegt.

Folgendes Begriffliche sei erläutert:
Ein "medizinisches Instrument" ist insbesondere jegliche mechanische oder mechanischelektrische Wirkeinheit, welche zur Diagnose und/oder Behandlung von Menschen oder Tieren geeignet ist. Das medizinische Instrument wird insbesondere zur Inspektion einer menschlichen oder einer tierischen Körperhöhle und/oder zur Manipulation von menschlichem oder tierischem Gewebe verwendet. Das medizinische Instrument weist insbesondere eine Trägereinheit zum Halten und/oder Handhaben des Instrumentes auf. Des Weiteren kann das medizinische Instrument eine Hohlsonde, einen Schaft, ein Werkzeug und/oder ein optisches System zur Betrachtung eines Sichtbereiches aufweisen. Das medizinische Instrument kann insbesondere ein Fasswerkzeug, ein Schneidwerkzeug, einen Nadelhalter, einen Clipsetzer und/oder ein andersartiges Werkzeug aufweisen. Bei einem medizinischen Instrument kann es sich beispielsweise um ein Endoskop mit einem langen Schaft und einem abwinkelbaren Endabschnitt oder um einen Lithotripter handeln. Bei dem medizinischen Instrument kann es sich um ein handgehaltenes und/oder handgeführtes Instrument oder um ein robotergestütztes Instrument handeln. Das medizinische Instrument und/oder die Trägereinheit kann insbesondere eine Zuführeinrichtung eines Druckmediums und/oder Fluides, wie beispielsweise Druckluft, eines Hydraulikfluides und/oder eines Wärmetransportmittels, aufweisen.

Eine "Trägereinheit" ist insbesondere ein Hand- und/oder Halteteil des medizinischen Instrumentes und/oder einer Lithotripsievorrichtung. Bei der Trägereinheit kann es sich insbesondere um eine Handhabe zur manuellen und/oder automatisierten Bedienung und/oder eine Verbindung des medizinischen Instrumentes handeln. Die Trägereinheit kann auch an einem distalen Ende eines Roboterarms angeordnet, verbunden und/oder automatisiert geführt sein. Die Trägereinheit weist insbesondere ein Gehäuse auf. Innenliegend weist die Trägereinheit mindestens einen ersten Innenraum auf, welcher mit einem Überdruck beaufschlagbar ist und/oder in welchem ein Überdruck entstehen kann. Die "Außenseite" der Trägereinheit ist insbesondere die Seite, welche zur Umgebung um die Trägereinheit und/oder das medizinische Instrument ausgerichtet ist. Somit ist die Außenseite der Trägereinheit die Seite außerhalb eines geschlossenen Raums der Trägereinheit.

Ein "Innenraum" ist insbesondere ein Raum innerhalb der Trägereinheit, welcher vor Einflüssen aus der Umgebung um die Trägereinheit und/oder des medizinischen Instrumentes geschützt ist. Bei dem Innenraum handelt es sich insbesondere um einen umbauten Raum innerhalb der Trägereinheit.

Unter einem "Überdruck" wird insbesondere ein Druck verstanden, welcher relativ zum Atmosphärendruck und/oder Luftdruck gemessen wird. Bei einem Überdruck handelt es sich insbesondere um einen Druck, welcher höher als der Atmosphärendruck und/oder Luftdruck ist. Bei einem Überdruck handelt es sich insbesondere um einen lokal erhöhten Druck innerhalb eines Innenraums der Trägereinheit. Ein Überdruck innerhalb des Innenraums kann insbesondere dadurch entstehen, dass zum gleichen Zeitpunkt ein größerer Volumenstrom eines Fluides in den Innenraum eingebracht als abgeführt wird. Ebenso kann ein Überdruck dadurch entstehen, dass ein vorhandener Raum verringert wird, ohne dass Material diesen Raum verlassen kann, beispielsweise wird bei Bewegung eins Projektils in einem Beschleunigungsrohr eines Lithotripters das Luftvolumen in Bewegungsrichtung komprimiert, sodass ein Überdruck entsteht. Für den Überdruck kann ein Sollwert vorgegeben werden, ab welchem mittels der Druckentlastungseinrichtung ein Freigeben der Aussparung und/oder des Dichtsitzes durch das mindestens eine elastische Dichtelement und somit eine Druckentlastung des beaufschlagten Innenraumes über den mindestens einen Druckentlastungskanal und die Aussparung in die Umgebung erfolgt. Prinzipiell kann der Überdruck durch die Zufuhr von jeglicher Art von Fluid in den Innenraum und/oder durch Kompression dieses Fluides auftreten.

Unter einer "Druckentlastungseinrichtung" wird insbesondere jegliche Einrichtung verstanden, mittels der ein druckbeaufschlagter Innenraum und/oder ein Bereich des Innenraumes druckentlastbar ist. Mittels der Druckentlastungseinrichtung wird insbesondere ein Überdruck und/oder ein Einwirken eines Fluides bei Verwendung des medizinischen Instrumentes und/oder der Trägereinheit in dem Körper eines Patienten verhindert. Die Druckentlastungseinrichtung weist insbesondere mindestens ein elastisches Dichtelement auf, welches in einer Aussparung in und/oder an der Außenseite der Trägereinheit angeordnet ist, wobei die Aussparung über zumindest einen Druckentlastungskanal mit dem druckbeaufschlagbaren Innenraum der Trägereinheit verbunden ist. Somit bildet die Druckentlastungseinrichtung insbesondere ein Sicherheits-, Überdruck- und/oder Rückschlagventil aus. Mittels der Druckentlastungseinrichtung wird ein Fluid im Innenraum über den zumindest einen Druckentlastungskanal, eine Austrittsöffnung des Druckentlastungskanal in die Aussparung und durch die Aussparung bei Freigeben der Austrittsöffnung und/oder der Aussparung durch das mindestens eine elastische Dichtelement in die Umgebung und somit in die Atmosphäre um die Trägereinheit und/oder das medizinische Instrument abgegeben. Dieses Ableiten des Fluides aus dem Innenraum in die Umgebung mittels der Druckentlastungseinrichtung erfolgt insbesondere bei Überschreiten eines vorgegebenen Anspruchsdruckes. Bei der Druckentlastungseinrichtung handelt es sich insbesondere um eine mittels des mindestens einen elastischen Dichtelements gewichtsbelastete und/oder mit Eigenspannung ausgebildete Druckentlastungseinrichtung. Die Druckentlastungseinrichtung ist insbesondere frei von einer Regelung. Somit ist die Druckentlastungseinrichtung eine selbsttätige Sicherheitseinrichtung gegen unerwünschte Drucküberschreitung.

Ein "Dichtelement" ist insbesondere ein Element, welches einen ungewollten Stoffübergang beidseitig des Dichtelementes verhindert, begrenzt oder zulässt. Bei einem Dichtelement handelt es sich insbesondere um ein statisches Dichtelement. Unter "elastisch" wird insbesondere die Eigenschaft des Dichtelementes verstanden, unter Krafteinwirkung seine Form zu verändern und bei Wegfallen der einwirkenden Kraft in die Ursprungsform zurückzukehren. Das elastische Dichtelement weist insbesondere Kunststoff und/oder ein Elastomer als formfesten, jedoch elastisch verformbaren Kunststoff auf. Das elastische Dichtelement kann insbesondere Naturkautschuk und/oder Silikonkautschuk aufweisen. Prinzipiell kann das elastische Dichtelement sämtliche Formen aufweisen und beispielsweise als elastisches Band, Schlauchelement, O-Ring oder X-Ring ausgebildet sein. Das elastische Dichtelement ist in seiner Form und/oder seinen weiteren Eigenschaften insbesondere derart ausgebildet, dass es die Aussparung vollständig oder zumindest teilweise belegt und/oder ausfüllt und somit im Normalbetrieb des medizinischen Instrumentes abdichtet, im kritischen Überdruckfall jedoch die Aussparung freigibt. Das Dichtelement ist insbesondere unter Krafteinwirkung und Zusammenpressen in der Aussparung eingebracht und/oder angeordnet, sodass es unter Spannung in der Aussparung vorliegt und durch seinen spannungsbehafteten, bündigen Dichtsitz den Hohlraum der Aussparung zumindest teilweise oder vollständig abdichtet. Im Falle des Freigebens löst sich das elastische Dichtelement insbesondere von dem Übergang zwischen dem Druckentlastungskanal und der Aussparung und/oder von einer Dichtfläche und/oder -linie in der Aussparung.

Bei einer "Aussparung" handelt es sich insbesondere um einen ausgesparten und somit einen freien Raum in und/oder an der Außenseite der Trägereinheit. Bei der Aussparung kann es sich insbesondere um eine Einkerbung, einen Einschnitt, eine Kerbe, ein Loch, eine Lücke, Nut, Vertiefung und/oder einen an der Oberseite offenen, geformten Hohlraum in der Außenoberfläche der Trägereinheit handeln.

Ein "Druckentlastungskanal" ist insbesondere eine Fluidverbindung, mittels welcher ein unerwünschter Überdruck aus einem Innenraum oder mehreren Innenräumen zu einer Aussparung oder mehreren Aussparungen ableitbar ist. Der Druckentlastungskanal kann prinzipiell jegliche Form aufweisen, beispielsweise ein im Querschnitt quadratischer oder runder Kanal, ein Rohr und/oder eine Bohrung. Der Druckentlastungskanal endet insbesondere mit einer Austrittsöffnung in der Aussparung.

In einer weiteren Ausführungsform des medizinischen Instrumentes weist die Druckentlastungseinrichtung ein zweites elastisches Dichtelement in einer zweiten Aussparung in und/oder an der Außenseite der Trägereinheit, ein drittes elastisches Dichtelement in einer dritten Aussparung in und/oder an der Außenseite der Trägereinheit und/oder optional ein weiteres elastisches Dichtelement in einer weiteren Aussparung in und/oder an der Außenseite der Trägereinheit auf.

Somit kann ein einziger Innenraum über zumindest einen Druckentlastungskanal und mehrere örtlich verteilte Aussparungen mit jeweils einem elastischen Dichtelement druckentlastet werden.

Ein zweites, drittes und/oder optional ein weiteres elastisches Dichtelement entspricht in seiner Funktion dem oben definierten Dichtelement, wobei die Dichtelemente jedoch unterschiedliche Eigenschaften und/oder Formen aufweisen können. Ebenso entspricht eine zweite, dritte und/oder optional eine weitere Aussparung der oben definierten Aussparung, wobei auch die Aussparungen unterschiedliche Abmessungen, Formen und/oder weitere Eigenschaften aufweisen können.

Um die Druckentlastungseinrichtung sicher und flexibel auszubilden und mehrere mit einem Überdruck beaufschlagte Innenräume zu entlasten, ist oder sind in der Trägereinheit ein zweiter Innenraum, ein dritter Innenraum, ein vierter Innenraum und/oder optional weitere Innenräume angeordnet, wobei der jeweilige Innenraum mit einem Überdruck beaufschlagbar und über jeweils einen Druckentlastungskanal mit der ersten Aussparung, der zweiten Aussparung, der dritten Aussparung und/oder optional mit der weiteren Aussparung verbunden ist.

Somit können mehrere Druckentlastungskanäle von mehreren verschiedenen überdruckbelasteten Innenräumen auf ein einziges Dichtelement in einer einzigen Aussparung wirken. Dadurch steht ein großer Entlüftungsquerschnitt aufgrund der mehreren Druckentlastungskanäle geführt zu der einzigen Aussparung mit einem elastischen Dichtelement zur Verfügung. Zudem ist die Konstruktion vereinfacht und kostengünstiger und aufgrund nur einer Aussparung wird Bauraum innerhalb der Trägereinheit eingespart, wenn mehrere Innenräume durch ein- und dasselbe elastische Dichtelement entlüftet werden. Somit dient die Druckentlastungseinrichtung mit nur einem einzigen elastischen Dichtelement in einer Aussparung als Sicherheitsventil für über mehrere Druckentlastungskanäle verbundene Innenräume der Trägereinheit. Neben der kostengünstigen Herstellung einer solchen Druckentlastungseinrichtung und der einfachen und schnellen Montage bei der Aufbereitung ist dadurch auch eine einfache Farbkennzeichnung und/oder eine farbliche Design-Gestaltung ermöglicht. Zudem muss bei der Wartung lediglich ein einziges Dichtelement in der einzigen Aussparung auf seine Funktionsfähigkeit kontrolliert werden.

Ebenso kann der zweite und jeder weitere Innenraum jedoch auch jeweils über einen Druckentlastungskanal separat mit einer zugehörigen einzelnen Aussparung verbunden sein. Dadurch können unterschiedliche Fluide mit jeweils einem Überdruck separat in die Umgebung abgeleitet werden und ein Rückfluss des Fluides in einen anderen Druckentlastungskanal und/oder Innenraum wird durch die getrennte Führung verhindert.

In einer weiteren Ausführungsform des medizinischen Instrumentes ist oder sind die erste Aussparung, die zweite Aussparung, die dritte Aussparung und/oder optional die weitere Aussparung jeweils mit zwei oder mehreren Druckentlastungskanälen verbunden.

Somit kann ein Überdruck aus unterschiedlichen Bereichen eines Innenraums oder aus mehreren unterschiedlichen Innenräumen jeweils über zwei oder mehrere Druckentlastungkanäle in eine Aussparung, jeweils zwei oder mehreren Aussparungen zugeführt werden.

Um im Normalbetrieb des medizinischen Instrumentes eine sichere Abdichtung und im kritischen Überdruckfall ein sicheres Freigeben der Aussparung zu gewährleisten, ist oder sind das elastische Dichtelement, das jeweilige elastische Dichtelement oder die elastischen Dichtelemente als elastisches Dichtband und/oder elastischer Dichtring ausgebildet.

Somit kann die Form des elastischen Dichtelementes spezifisch in Abhängigkeit von der Form der jeweiligen Aussparung und/oder umgekehrt gewählt werden. Im Falle einer rechteckigen Aussparung und einem elastischen Dichtelement mit rechteckigem Querschnitt ausgebildet als Dichtband können dadurch mit dem Dichtband mehrere Innenräume kontrolliert werden.

In einer weiteren Ausführungsform des medizinischen Instrumentes ist oder sind die Aussparung, die jeweilige Aussparung oder die Aussparungen an ihrer Seitenwand oder ihren Seitenwänden konisch geformt.

Dadurch wird eine definierte Auflage- und Dichtfläche für das elastische Dichtelement innerhalb der Aussparung bereitgestellt, an welcher das Dichtelement unter Spannung anordenbar ist. Dadurch, dass sich die Aussparung beispielsweise in Richtung zum Druckentlastungskanal konisch verengt, ist das Dichtelement in einfacher Weise in die Aussparung einsetzbar und/oder einpressbar. Dagegen erleichtert und beschleunigt im Entlastungsfall die konische Erweiterung der Aussparung zur Außenseite der Trägereinheit ein Lösen und Abheben des elastischen Dichtelementes von der Dichtfläche oder -linie an der jeweiligen Seitenwand.

Bevorzugt wird eine konisch geformte Aussparung mit einem Dichtring als elastisches Dichtelement nur bei der Verbindung mit einem Innenraum eingesetzt, da ansonsten bei Verbindung mit mehreren Innenräumen die Gefahr besteht, dass diese kommunizieren und der Überdruck von einem Innenraum zu einem anderen Innenraum übertragen werden könnte, ohne dass der Überdruck nach außen entweicht. Deshalb wird bei konischen Seitenwänden der Aussparung, in denen ein Dichtring sitzt, bevorzugt für jeden Innenraum eine separate Aussparung in und/oder an der Außenseite des Trägerelementes bereitgestellt und jeweils mit einem separaten Dichtring bestückt. Somit wird jeweils eine speziell geformte Aussparung mit konischen Flanken und/oder einer Auflagelinie für den Dichtring bereitgestellt.

Um jeweils gezielt den Bereich in der Aussparung um die jeweilige Austrittsöffnung des Druckentlastungskanals abzudichten, kann oder können die Aussparung, die jeweilige Aussparung oder die Aussparungen eine geformte Auflagefläche für das jeweilige elastische Dichtelement aufweisen.

Bei der "Auflagefläche" handelt es sich insbesondere um eine spezifisch geformte Fläche und/oder einen Abschnitt in und/oder an der Aussparung. Bei der Auflagefläche kann es sich auch um einen Dichtsitz handeln, sodass durch die geformte Auflagefläche gleichzeitig eine definierte Dichtfläche und/oder -linie bereitgestellt wird. Die Auflagefläche kann als formschlüssig passendes Gegenstück zum Dichtelement und/oder einem Teil des Dichtelementes ausgebildet sein. Bei der Auflagefläche kann es sich beispielsweise um einen konzentrischen Ring um die Austrittsöffnung des Druckentlastungskanals handeln, wobei das beispielsweise als Dichtring ausgebildete elastische Dichtelement mit seiner Unterseite auf der Oberseite des Ringes aufliegt und somit die Austrittsöffnung bedeckt. In Abhängigkeit der Form und/oder Größe der Dichtfläche und/oder -linie und des Gewichtes und der Elastizität des jeweiligen Dichtelementes ist der Ansprechdruck einstellbar, bei dem im Falle eines kritischen Überdruckes das Dichtelement sich verformt und/oder bewegt und folglich durch Abheben von der Auflagefläche, Dichtfläche und/oder -linie die Austrittsöffnung des Druckentlastungskanals und/oder den dichtenden Bereich der Aussparung freigibt. Durch mehrere speziell geformte Auflageflächen in einer einzigen Aussparung können zwei oder mehrere Innenräume in derselben Aussparung entlüftet werden.

In einer weiteren Ausführungsform des medizinischen Instrumentes weist oder weisen die Aussparung, die jeweilige Aussparung oder die Aussparungen eine Dichtrippe oder zwei oder mehrere Dichtrippen auf.

Dadurch kann durch jede Austrittsöffnung des Druckentlastungskanals umgebenden Dichtrippen im Falle der Verbindung von mehreren Innenräumen mit einer einzigen Aussparung ein Überströmen des Fluids von einem Innenraum in den anderen Innenraum verhindert werden, da die Dichtrippen jeweils umlaufende separate Dichtlinien ausbilden. Hierbei sind die jeweiligen Dichtrippen so angeordnet, dass diese vollständig eine Austrittsöffnung des jeweiligen Druckentlastungskanals umgeben und mit ihrer Oberseite gleichzeitig eine Auflagefläche und somit Dichtfläche für das elastische Dichtelement ausbilden.

In einer Ausführungsform, in der ein Dichtband auf mehreren Dichtrippen aufliegt, können die Dichtrippen im mittleren Bereich der Aussparung so angeordnet sein, dass der äußere Bereich der Aussparung frei von Dichtrippen ist, wodurch eine höhere Beweglichkeit am Außenrand des Dichtbandes vorliegt und dadurch die Entlüftung bevorzugt am Außenrand des Dichtbandes erfolgt und nicht zur Mitte hin und/oder in Richtung der anderen Innenräume.

Eine "Dichtrippe" ist insbesondere ein plattenförmiges Bauteil und/oder eine Platte. Die Dichtrippe ist insbesondere senkrecht stehend auf der Unterseite der Aussparung um und/oder neben der Austrittsöffnung des Druckentlastungskanals in die Aussparung angeordnet. Insbesondere können mehrere Dichtrippen um die Austrittsöffnung angeordnet sein. Die Dichtrippe ist insbesondere stoffschlüssig mit der Innenfläche der Aussparung verbunden.

Um einen Wärmeabtransport aus dem medizinischen Instrument und/oder einen Abtransport von Steinmaterial zu ermöglichen, kann das medizinische Instrument eine Fluiddurchführung zum Abtransportieren eines zertrümmerten Steinfragmentes und/oder zum Wärmetransport aufweisen.

In einer weiteren Ausführungsform ist das medizinische Instrument eine ballistische Lithotripsievorrichtung, insbesondere eine ballistische intrakorporale Lithotripsievorrichtung, zum Zertrümmern von Körpersteinen, wobei die Lithotripsievorrichtung ein Führungsrohr mit einem Hohlraum, mit einem proximalen Ende und mit einem distalen Ende, ein bewegbares Projektil, ein distalseitiges Anschlagselement und ein proximalseitiges Anschlagselement für das bewegbare Projektil aufweist, wobei das Führungsrohr zumindest teilweise in der Trägereinheit angeordnet ist, und der Lithotripsievorrichtung eine Antriebseinrichtung zum Hin- und Herbewegen des Projektils zwischen dem distalseitigen Anschlagselement und dem proximalseitigen Anschlagselement und eine Sonotrode zuordenbar sind, und die Sonotrode an ihrem proximalen Ende mit der Trägereinheit und/oder dem Führungsrohr direkt oder indirekt verbindbar und durch ein mechanisches Auftreffen des Projektils auf das distalseitige Anschlagselement schwingungsanregbar ist, wobei in einem Fehlerfall der jeweilige Innenraum mit dem Überdruck aufgrund einer Kompression in dem Hohlraum des Führungsrohrs durch das Hin- und Herbewegen des Projektils beaufschlagbar ist.

Somit wird eine ballistische Lithotripsievorrichtung bereitgestellt, bei der im Fehlerfall ein Überdruck in dem komprimierten Luft- und/oder Gasvolumen in dem Hohlraum des Führungsrohres bedingt durch das Hin- und Herbewegen des Projektils gezielt mittels der Druckentlastungseinrichtung nach außen in die Umgebung der Lithotripsievorrichtung abgeführt wird und nicht in den Körper des Patienten gelangen kann.

Bei einer "Lithotripsievorrichtung" (auch "Lithotripter" genannt) handelt es sich insbesondere um eine Vorrichtung zum Zertrümmern von Körpersteinen durch Stöße, Stoßwellen und/oder Verformungswellen. Unter einer Lithotripsievorrichtung werden insbesondere verschiedene Bestandteile, Bau- und/oder Funktionskomponenten eines Lithotripters verstanden. Die Lithotripsievorrichtung kann einen Lithotripter vollständig oder teilweise ausbilden. Bei einer Lithotripsievorrichtung kann es sich insbesondere um eine intrakorporale oder extrakorporale Lithotripsievorrichtung handeln. Im Falle einer intrakorporalen Lithotripsievorrichtung kann diese zusätzlich eine Spül-/Saugpumpe aufweisen. Die Lithotripsievorrichtung kann als Handgerät ausgebildet sein und/oder ein Endoskop aufweisen oder in ein Endoskop eingeschoben werden. Die Lithotripsievorrichtung ist insbesondere autoklavierbar und weist beispielsweise Instrumentenstahl und/oder Kunststoff auf. Die Lithotripsievorrichtung kann weitere Komponenten, wie ein Steuer- und/oder Versorgungsgerät aufweisen oder diese sind der Lithotripsievorrichtung zugeordnet.

Bei der "ballistischen" Lithotripsievorrichtung erfolgt durch Hin- und Herbewegen des Projektils und dem distalseitigen Aufschlag des Projektils an dem distalseitigen Anschlagselement eine Stoßanregung der Sonotrode. Hierbei tritt eine ballistische Bewegung auf, welche schnell und kurzdauernd ist und deren Verlauf nicht oder nur geringfügig veränderbar ist. Bei einer "pneumatischen" Lithotripsievorrichtung wird Druckluft und/oder ein Druckgas zum Antrieb und somit zur ballistischen Bewegung des Projektils verwendet.

Unter "Körpersteinen" (auch "Konkrement" genannt) werden insbesondere alle Steine in einem menschlichen oder tierischen Körper verstanden, welche sich z.B. aus Salzen und Eiweißen durch Kristallisation und/oder Kondensation bilden. Bei Körpersteinen kann es sich beispielsweise um Gallensteine, Harnsteine, Nierensteine und/oder Speichelsteine handeln.

Ein "Projektil" ist insbesondere ein Körper, welcher innerhalb eines Hohlraums eines Führungsrohres (auch Beschleunigungsrohr genannt) einer Lithotripsievorrichtung frei entlang einer Beschleunigungsstrecke beweglich ist. Das Projektil ist insbesondere zwischen einem proximalseitigen Anschlagselement und einem distalseitigen Anschlagselement innerhalb des dazwischen angeordneten Hohlraums des Führungsrohrs hin- und zurückbewegbar. Das Projektil kann auch innerhalb des Führungsrohres von einer Steuerhülse umgeben sein. Prinzipiell kann das Projektil jegliche Form aufweisen. Beispielsweise kann das Projektil die Form eines Bolzens oder einer Kugel aufweisen. Das Projektil weist insbesondere harten Stahl und/oder magnetische Eigenschaften auf. Für die freie Beweglichkeit weist das Projektil insbesondere einen etwas geringeren Außendurchmesser als der Durchmesser des Hohlraums des Führungsrohrs und/oder der Steuerhülse auf. Beispielsweise kann das Projektil einen Außendurchmesser von 8 mm, bevorzugt von 6 mm, aufweisen. Das Projektil kann insbesondere zwischen dem proximalseitigen Anschlagselement und dem distalseitigen Anschlagselement und somit entlang einer Beschleunigungsstrecke stetig beispielsweise mittels eines Druckmediums der Antriebseinrichtung hin- und/oder herbewegt werden. Bevorzugt wird das Projektil kontinuierlich intermittierend und/oder oszillierend zwischen dem proximalseitigen Anschlagselement und dem distalseitigen Anschlagselement hin- und herbewegt.

Bei einer "Antriebseinrichtung" kann es sich prinzipiell um jegliche Art von Einrichtung handeln, welche eine Kraft auf das Projektil und somit eine Bewegung des Projektils bewirkt. Bei der Antriebseinrichtung kann es sich beispielsweise um eine Vorrichtung handeln, welche mittels Lasers, eines Druckmediums, beispielsweise pneumatisch mithilfe von Druckluft, mittels eines elektromagnetischen Feldes und/oder mittels einer mechanischen Vorrichtung das Projektil beschleunigt. Eine Antriebseinrichtung kann insbesondere mittels eines Zuführens und/oder Abführens eines Druckmediums eine Kraft auf das Projektil und somit eine Bewegung des Projektils bewirken. Die Antriebseinrichtung ermöglicht insbesondere ein kontinuierliches und gleichmäßiges Einströmen des Druckmediums durch proximalseitige und distalseitige Durchgangsöffnungen des Führungsrohrs und proximalseitige und distalseitige Öffnungen der Steuerhülse und eine Beschleunigung des Projektils innerhalb des Hohlraums der Steuerhülse und/oder des Führungsrohrs.

Unter "distalseitig" und "distal" wird eine patientenkörpernahe und somit benutzerferne Anordnung und/oder ein entsprechendes Ende oder Abschnitt verstanden. Dementsprechend wird unter "proximalseitig" oder "proximal" eine benutzernahe und somit körperferne Anordnung oder ein entsprechendes Ende oder Abschnitt verstanden.

Eine "Sonotrode" (auch "Sonde" genannt) ist insbesondere ein Bauteil, welches durch Einwirken und/oder Einleiten von mechanischen Schwingungen selbst in Schwingung, Resonanzschwingung und/oder Verformungsschwingung versetzt wird. Eine Sonotrode weist insbesondere ein Kopfstück (auch "Nippel", "Haltenippel" oder "Grundkörper" genannt) und ein längliches Einführteil, beispielsweise ein Sondenrohr oder Sondenstab, auf. Das Einführteil ist insbesondere in einer Aufnahmeeinheit in dem dickerem Kopfstück aufgenommen. Bei der Aufnahmeeinheit handelt es sich beispielsweise um eine Bohrung im Kopfstück, in welcher das proximale Ende und/oder der proximale Endabschnitte des Einführteils fest und/oder unlösbar gefügt, beispielweise verlötet, ist. Bei einer Sonotrode handelt es sich insbesondere um ein längliches Bauteil. Eine Sonotrode ist beispielsweise zumindest teilweise stab-, röhren- und/oder schlauchförmig ausgebildet. Die Sonotrode kann eine Hohlsonde sein. Die Sonotrode kann einstückig oder mehrteilig ausgebildet sein. Die Sonotrode weist im Sondenrohr insbesondere einen Durchmesser in einem Bereich von 0,5 mm bis 4,5 mm, insbesondere von 0,8 mm bis 3,8 mm, auf. Die Sonotrode weist insbesondere Stahl-, Eisen-, Cobalt-, Chrom-, Nickel-, Molybdän-, Titan-, Magnesium- und/oder Aluminiumlegierungen und/oder Carbon- oder Glasverbundwerkstoffe auf. Mittels der Stoßenergie beim Anschlagen des Projektils an dem distalseitigen Anschlagselement und/oder einem proximalen Ende einer Federeinrichtung mit Zwischenspeichern und Übertragen der Bewegungsenergie wird insbesondere der Sonotrode eine gezielt geformte Verformungswelle aufgeprägt. Die Verformungswelle bewirkt insbesondere eine translatorische Bewegung des distalen Sonotrodenendes, welche aufgrund der großen Auslenkung eine verbesserte Steinzertrümmerung bewirkt. Neben dem mechanischen Stoß kann die Sonotrode zusätzlich insbesondere mittels einer Schwingungsanregungseinrichtung, beispielsweise mit einem Ultraschallschwingungsanreger, in eine Schwingung, insbesondere longitudinale Schwingung, angeregt werden. Somit ist die Sonotrode insbesondere als Wellenleiter für die Schwingungswellen erzeugt von einer Schwingungsanregungseinrichtung und/oder für die Stoßwellen und/oder Verformungswellen des Projektils ausgebildet. Das proximale Ende der Sonotrode kann insbesondere direkt oder indirekt am distalen Anschlagselement anliegen. Bevorzugt ist das Kopfstück der Sonotrode beweglich gelagert. Die Sonotrode ist insbesondere derart geformt, dass diese optimal die Schwingungswellen, Verformungswellen, Stoßwellen und/oder die Ultraschallschwingung an ihrem distalen Ende in den Körper, die zu behandelnde Körperregion und/oder direkt auf den zu zertrümmernden Körperstein einleitet. Vorliegend sei herausgestellt, dass in der Lithotripsievorrichtung, in welcher die Druckentlastungseinrichtung verwendet wird, üblicherweise nicht Stoßwellen, sondern Verformungswellen auftreten, da in dieser Lithotripsievorrichtung nicht eine Schnelle und/oder Teilchengeschwindigkeit vorliegt, die schneller als der Schall ist (in Metallen circa 5.000 m/s).

In einer weiteren Ausführungsform ist das medizinische Instrument eine pneumatische Lithotripsievorrichtung, und mittels der Antriebseinrichtung ist ein Druckmedium in ein Inneres der Trägereinheit und/oder des Führungsrohrs zum Hin- und Herbewegen des Projektils in den Hohlraum des Führungsrohres zu- und/oder abführbar, wobei in einem Fehlerfall mittels des Druckmediums der jeweilige Innenraum mit einem Überdruck beaufschlagbar ist.

Somit wird aktiv ein Druckmedium, wie Druckluft und/oder ein Druckgas, im Falle einer pneumatischen Lithotripsievorrichtung dem Hohlraum des Führungsrohres zum Bewegen des Projektils zugeführt. Dadurch besteht eine erhöhte Gefahr, dass aufgrund eines Bedien- und/oder Funktionsfehlers ein durch das Druckmedium bedingter Überdruck entsteht, welcher betriebssicher mittels der Druckentlastungseinrichtung in die äußere Umgebung der pneumatischen Lithotripsievorrichtung abgegeben werden kann.

In einer weiteren Ausführungsform ist die Sonotrode eine Hohlsonde.

Im Falle der Ausbildung der Sonotrode als Hohlsonde und im Fehlerfall kann prinzipiell direkt das Druckmedium vom Inneren der Trägereinheit durch die Hohlsonde bis in den Körper des Patienten gelangen. Durch die erfindungsgemäße Druckentlastungseinrichtung wird dieses Risiko verhindert oder zumindest deutlich minimiert.

Die Zeichnungen, die Beschreibung und die Ansprüche enthalten zahlreiche Merkmale in Kombination. Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Im Weiteren wird die Erfindung anhand von Ausführungsbeispielen näher erläutert. Es zeigen
- Figur 1: eine stark schematische dreidimensionale Darstellung einer Lithotripsievorrichtung,
- Figur 2: eine stark schematische Darstellung der Lithotripsievorrichtung mit einem Führungsrohr und einer Steuerhülse in Längsrichtung bei einer Bewegung des Projektils in distaler Richtung mit einem Überdruckventil,
- Figur 3: eine stark schematische Darstellung des distalen Bereichs der Lithotripsievorrichtung aus Figur 2 mit dem Überdruckventil,
- Figur 4: eine stark schematische Darstellung eines Überdruckventils mit einem Dichtring in einer konischen Aussparung einer Trägereinheit im Längsschnitt,
- Figur 5: eine Alternative des Überdruckventils mit einem Dichtring teilweise in einer konischen Aussparung einer Trägereinheit im Längsschnitt,
- Figur 6: eine stark schematische Darstellung des Überdruckventils aus Figur 3 mit einem Elastomer-Band in einer rechteckigen Aussparung der Trägereinheit im Längsschnitt,
- Figur 7: eine weitere Alternative des Überdruckventils mit einem Elastomer-Band und Dichtrippen in einer rechteckigen Aussparung einer Trägereinheit im Längsschnitt, und
- Figur 8: eine weitere Alternative des Überdruckventils mit einem Elastomer-Band und Dichtrippen in einer rechteckigen Aussparung verbunden mit zwei Entlastungsbohrungen einer Trägereinheit im Längsschnitt.

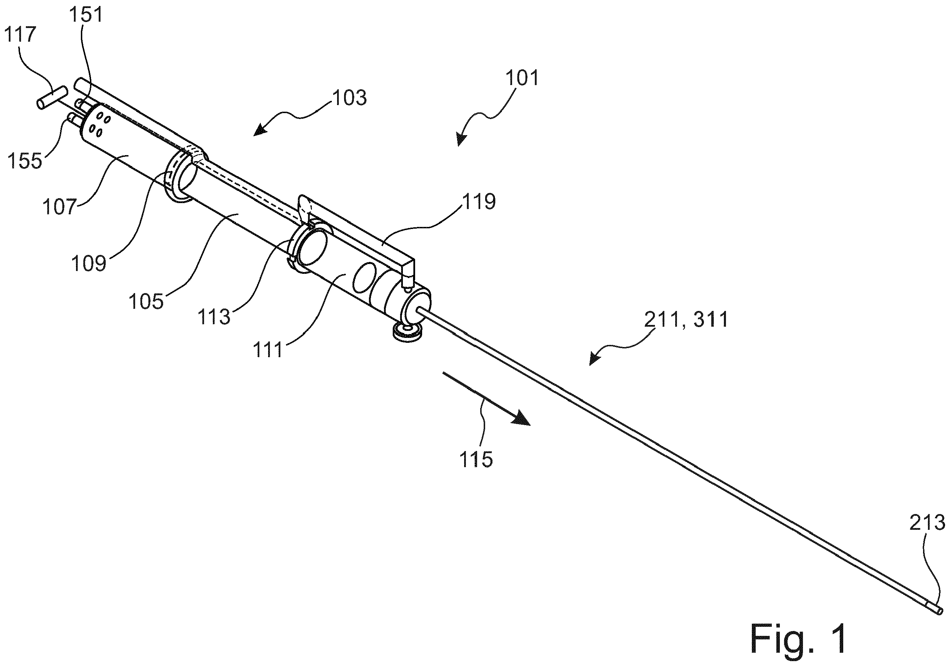

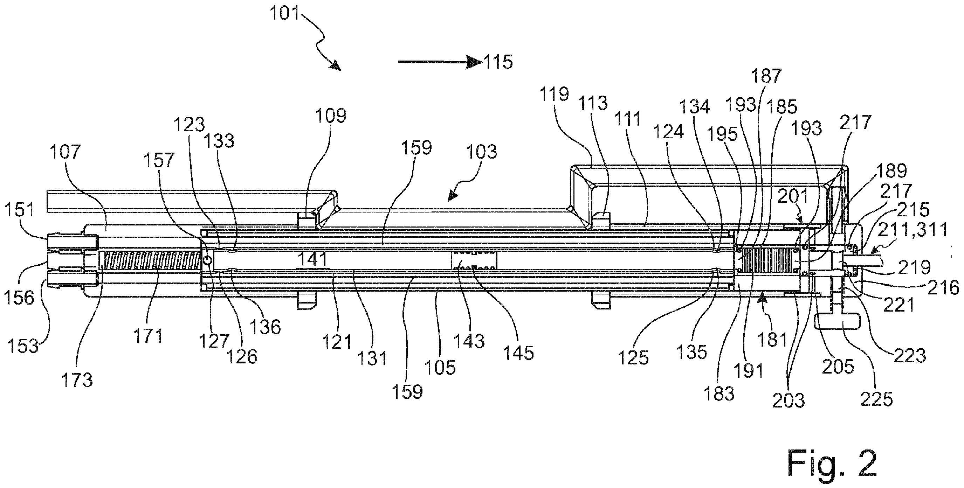

Eine Lithotripsievorrichtung 101 weist eine Trägereinheit 103 mit einem mittigen Gehäuserohr 105 auf. An einem proximalen Ende des Gehäuserohrs 105 ist eine proximale Endkappe 107 mittels einer proximalen Gegenmutter 109 auf das Gehäuserohr 105 aufgeschraubt. Ebenso ist am distalen Ende des Gehäuserohrs 105 eine distale Endkappe 111 mittels einer distalen Gegenmutter 113 aufgeschraubt (siehe Figur 1 und Figur 2). Am proximalen Ende der proximalen Endkappe 107 ist ein erster Abluftanschluss 151 und ein in Figur 1 nicht sichtbarer zweiter Abluftanschluss 153 angeordnet. Des Weiteren ist proximalseitig der proximalen Endkappe 107 ein erster Zuluftanschluss 155 und ein nicht in Figur 1 sichtbarer zweiter Zuluftanschluss 156 angeordnet. Vom distalen Endabschnitt der distalen Endkappe 111 ist eine Absaugleitung 119 zum Absaugen von Körpersteinbruchstücken entgegen einer distalen Richtung 115 zum proximalen Ende der Lithotripsievorrichtung 101 geführt. Die Absaugleitung 119 ist in der Figur 1 lediglich symbolisch dargestellt und weist nicht praktikable enge Biegeradien auf. Ebenso ist in der Figur 1 ein Bedienelement 117 am proximalen Ende der Trägereinheit 103 lediglich symbolisch dargestellt, wobei das Bedienelement 117 in einer alternativen Ausgestaltung optimal ergonomisch am Gehäuserohr 105 angeordnet ist. Das Bedienelement 117 ist mit einer innen im Gehäuserohr 105 angeordneten Steuerhülse 131 zum Starten und Ausschalten sowie für einen Einzel- und/oder Dauerbeschuss mittels der ballistischen Lithotripsievorrichtung 101 ausgelegt. Am distalen Ende der Trägereinheit 103 ist eine langgestreckte als Hohlsonde 311 ausgebildete Sonotrode 211 mit einer Sonotrodenspitze 213 angeordnet.

Im Inneren des Gehäuserohrs 105 der Trägereinheit 103 ist ein Führungsrohr 121 beabstandet zum Gehäuserohr 105 angeordnet, wobei zwischen einer Innenwand des Gehäuserohrs 105 und einer Außenwand des Führungsrohrs 121 jeweils zwei symmetrisch über den Querschnitt angelegte Zuluftkammern 157 und Abluftkammern 159 angeordnet sind, welche über Bohrungen in der proximalen Endkappe 107 mit den über Kreuz angeordneten ersten Abluftanschluss 151 und dem zweiten Abluftanschluss 153 sowie dem ersten Zuluftanschluss 155 und dem zweiten Zuluftanschluss 156 verbunden sind (in Figur 2 liegt die eine Zuluftkammer 157 hinter einer fünften Durchgangsbohrung 127 und ist nur durch diese sichtbar, während die zweite Zuluftkammer vor der Betrachtungsebene liegt). Die Zuluftkammern 157 und die Abluftkammern 159 erstrecken sich über die gesamte Länge des Führungsrohrs 121. Die beiden Abluftanschlüsse 151, 153 und die beiden Zuluftanschlüsse 155, 156 sind jeweils über ein nicht gezeigtes Y-Verbindungsstück mit einem Abluftschlauch und einem Zuluftschlauch einer nicht gezeigten Antriebsvorrichtung verbunden.

Das Führungsrohr 121 weist proximalseitig eine erste Durchgangsbohrung 123 und eine vierte Durchgangsbohrung 126 auf, welche jeweils mit einer der beiden Abluftkammern 159 verbunden sind. Distalseitig weist das Führungsrohr 121 eine zweite Durchgangsbohrung 124 und eine dritte Durchgangsbohrung 125 auf, welche jeweils mit einer der beiden Abluftkammern 159 verbunden sind. Des Weiteren weist das Führungsrohr 121 proximalseitig die fünfte Durchgangsbohrung 127 und eine gegenüberliegende und deshalb in Figur 2 nicht sichtbare weitere Durchgangsbohrung auf, welche jeweils mit einer der beiden Zuluftkammern 157 verbunden sind (die beiden entsprechenden distalseitigen Durchgangsbohrungen verbunden mit den Zuluftkammern 157 sind in Figur 2 nicht gezeigt). Alle Durchgangsbohrungen 123, 124, 125, 126, 127 gehen jeweils quer durch die Mantelfläche des Führungsrohrs 121 durch und weisen einen Durchmesser von jeweils 3 mm auf.

Innenliegend in dem Führungsrohr 121 ist die Steuerhülse 131 angeordnet, welche proximalseitig korrespondierend zu den proximalseitigen Durchgangsbohrungen 123, 126 des Führungsrohrs 121 eine erste Ventilbohrung 133 und eine vierte Ventilbohrung 136 aufweist. Dementsprechend ist distalseitig eine zweite Ventilbohrung 134 und eine dritte Ventilbohrung 135 in der Steuerhülse 131 eingebracht. Die Steuerhülse 131 ist verdrehsicher innerhalb des Führungsrohres 121 angeordnet, sodass die korrespondierenden Durchgangsbohrungen des Führungsrohrs 121 und die Ventilbohrungen der Steuerhülse 131 in einer jeweiligen Ventilöffnungsstellung frei durchgängig sind. Die Steuerhülse 131 weist innenliegend einen Hohlraum 141 auf, welcher gleichzeitig eine Beschleunigungsstrecke für ein Projektil 143 ausbildet. Das Projektil 143 weist an seiner Außenoberfläche einen Mitnehmerring 145 auf, welcher außen an einer Innenfläche der Steuerhülse 131 anliegt. Die Steuerhülse 131 ist um 4 mm kürzer als das Führungsrohr 121.

Proximalseitig von der Steuerhülse 131 ist eine Rückholfeder 171 in einem Hüllrohr angeordnet, welche in der proximalen Endkappe 107 mittels einer Halterung 173 gehalten ist.

Am distalen Ende der Steuerhülse 131 ist eine Tempierfeder 181 zum Aufprägen einer definierten Verformungswelle auf die Sonotrode 211 aufgrund des mechanischen Stoßes des Projektils 143 angeordnet. Die Tempierfeder 181 weist eine Vielzahl von gestapelten Polymer-Scheiben 191 in distaler Richtung 115 auf, welche außen von einem Hüllrohr 185 umgeben sind. Das Hüllrohr 185 ist mittels eines Halters 183 in der distalen Endkappe 111 gehalten. Am proximalen Ende des Hüllrohrs 185 ist eine proximale Abschlusskappe 187 angeordnet, welche innenliegend einen O-Ring 193 aufweist und mittels eines Schweißrings 195 beweglich gefangen und gefasst ist, welcher mit dem Hüllrohr 185 verschweißt ist. Distalseitig ist eine distale Abschlusskappe 189 angeordnet, welche mittels einer Umbördelung des Hüllrohrs 185 ebenfalls beweglich gefasst ist und ebenfalls innenliegend einen O-Ring 193 aufweist.

Somit stellt das distale Ende der Rückholfeder 171 ein proximales Anschlagselement und die proximale Abschlusskappe 187 der Tempierfeder 181 ein distalseitiges Anschlagselement für das Projektil 143 dar. Die Figur 2 zeigt den Zustand, bei dem die Steuerhülse 131 in einer distalen Richtung 115 gegen das distalseitige Anschlagselement ausgebildet durch die proximale Abschlusskappe 187 der Tempierfeder 181 angeschlagen ist. Da die Steuerhülse 131 um 4 mm kürzer als das Führungsrohr 121 ist, ist der Hohlraum des Führungsrohres 121 im Bereich der proximalseitigen fünften Durchgangsöffnung 127 frei von der Steuerhülse 131. Nach Repulsation des Projektils 143 an der Abschlusskappe 187 der Tempierfeder 181 bewegt sich das Projektil 143 zurück entgegen der distalen Richtung 115 und nimmt mittels des Mitnehmerringes 145 die Steuerhülse 131 mit. Durch diese Rückbewegung werden die in Figur 2 nicht gezeigten gegenüberliegenden distalseitigen Durchgangsöffnungen des Führungsrohres 121 und die zugehörigen Ventilöffnungen für den Eintritt von Zuluft unter Druck in den Hohlraum 141 der Steuerhülse 131 frei durchgängig und die eintretende Zuluft drückt das Projektil 143 weiter in die proximale Richtung. Aufgrund dieser Zurückbewegung des Projektils 143 und der Mitnahme der Steuerhülse 131 wird bei einem Weg von 4 mm die erste Ventilbohrung 133 der Steuerhülse 131 auf die erste Durchgangsbohrung 123 des Führungsrohres 121 und die vierte Ventilbohrung 136 der Steuerhülse 131 auf die vierte Durchgangsbohrung 126 des Führungsrohres 121 bei einem definierten Anschlag des proximalen Endes der Steuerhülse 131 gegen die distalseitige Stirnwand des Hüllrohres der Rückholfeder 171 geschoben, wodurch die jeweilige Durchgangsbohrung und Ventilbohrung durchgängig für den Austritt von Abluft in die Abluftkammern 159 sind. Gleichzeitig wird die fünfte Durchgangsbohrung 127 und die gegenüberliegende nicht sichtbare weitere Durchgangsbohrung für den Durchtritt von Zuluft geschlossen. Nach Repulsation des Projektils 143 am proximalen Anschlag mittels der Rückholfeder 171 und gefolgt von erneutem Hinbewegen in distaler Richtung 115 wird die Steuerhülse 131 mittels des Mitnehmerringes 145 erneut vom Projektil 143 mitgenommen und dadurch werden die fünfte Durchgangsbohrung 127 und die gegenüberliegende nicht sichtbare weitere Durchgangsbohrung geöffnet. Durch diese tritt Zuluft in den Hohlraum 141 der Steuerhülse 131 ein und bewegt das Projektil 143 weiter in distaler Richtung 115, bis der in Figur 2 gezeigte Zustand wieder erreicht ist.

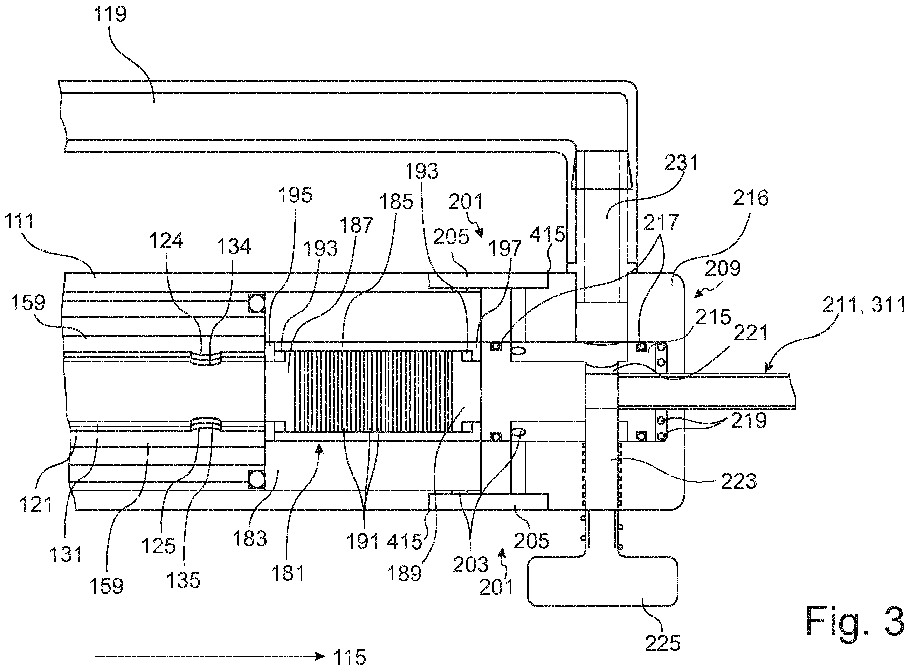

Distalseitig der Tempierfeder 181 ist eine Kopfvorrichtung 209 mit einem Kopfstück 215 der Sonotrode 211 angeordnet, wobei das Kopfstück 215 an seinem proximalen Ende und seinem distalen Ende jeweils mittels O-Ringen 217 beweglich in einem Führungsteil 216 gelagert ist. Das Kopfstück 215 weist eine Querbohrung 221 auf, in welcher ein Entstopfungsstö-ßel 223 als Verdrehsicherung und zum Entfernen von Körpersteinbruchstücken mit einem Betätigungsgriff 225 lose eingreift (siehe Figuren 2 und 3). Der Entstopfungsstößel 223 wird mittels einer nicht in den Figuren 2 und 3 gezeigten Feder in Position gehalten. Distalseitig am Kopfstück 215 ist ein Bremselement 219 zur Begrenzung einer Amplitude der Sonotrode 211 angeordnet.

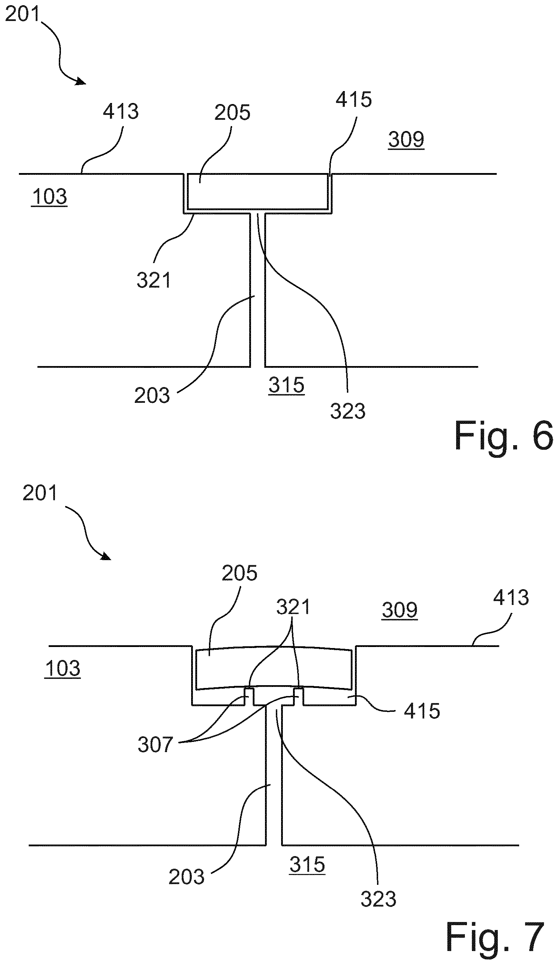

In der distalen Endkappe 111 sind Entlastungsbohrungen 203 zum Kopfstück 215 der Sonotrode 211 und zum Halter 183 der Tempierfeder 181 eingebracht, welche gemeinsam mit einem jeweiligen Elastomer-Band 205 in einer jeweiligen rechteckigen Aussparung 415 in einer Außenseite der Trägereinheit 103 jeweils ein Überdruckventil 201 zum Halter 183 der Tempierfeder 181 und zum Kopfstück 215 der Sonotrode 211 ausbilden (Figur 3), um ein Einwirken eines Überdruckes in dem Patienten bei Verwendung der Lithotripsievorrichtung 101 bei Auftreten eines Fehlers zu verhindern. Eines der beiden Überdruckventil 201 ist stark vereinfacht in der Figur 6 dargestellt. In der Außenwand 413 der Trägereinheit 103 ausgerichtet zur Umgebung 309 ist die rechteckige Aussparung 415 angeordnet, in welcher das Elastomer-Band 205 unter Spannung eingelegt worden ist und dadurch an den innenliegenden Seitenwänden der rechteckigen Aussparung 415 einen rundumlaufenden Dichtsitz aufweist. An seiner Außenseite zur Umgebung 309 schließt das Elastomer-Band 205 bündig mit der Außenwand 413 der Trägereinheit 103 ab. An der Unterseite des Elastomer-Bandes 205 liegt dieses auf einer ebenfalls dichtenden Auflagefläche 321 der rechteckigen Aussparung 415 auf. Das Elastomer-Band 205 dichtet mittig mit seiner Unterseite eine Austrittsöffnung 323 der Entlastungsbohrung 203 ab, welche mit einem Innenraum 315 der Lithotripsievorrichtung 101 verbunden ist.

Ein Funktionsfehler beim Betrieb der Lithotripsievorrichtung 101 unter kontinuierlicher Zuführung von Druckluft in die Steuerhülse 131 kann beispielsweise durch Ermüdung oder einen Montagefehler bei der Aufbereitung des proximalseitigen O-Ringes 193 des Hüllrohres 185 der Tempierfeder 181 und der O-Ringe 217 des Kopfstückes 215 auftreten. Dann erfolgt ein unerwünschtes Übertreten der Druckluft von der Steuerhülse 131 in die Tempierfeder 181 und weiter in das Kopfstück 215 der Sonotrode 211. Dadurch liegt im Halter 183 der Tempierfeder 181 und im Kopfstück 215 ein kritischer Überdruck vor. Die kritische Druckluft strömt aus dem Halter 183 und Kopfstück 215 (jeweils Innenraum 315) durch jeweils eine Entlastungsbohrung 203 und die jeweilige Austrittsöffnung 323 in die jeweilige rechteckige Aussparung 413, wodurch sich das jeweilige Elastomer-Band 205 aus der rechteckigen Aussparung 415 löst und angehoben wird und dadurch ein Austritt der kritischen Druckluft in die Umgebung 309 erfolgt. Durch die beiden Überdruckventile 201 wird die kritische Druckluft in der Tempierfeder 181 und dem Kopfstück 215 redundant und sicher in die Umgebung 309 abgeführt und kann nicht durch den Hohlraum der als Hohlsonde 311 ausgebildeten Sonotrode 211 in den Körper eines Patienten bei Einwirken der Sonotrodenspitze 213 während des Zertrümmerns eines Körpersteines gelangen und dort gegebenenfalls Schäden im Körper aufgrund des Überdruckes und/oder einer Kontamination verursachen.

In einer in Figur 7 gezeigten Alternative des Überdruckventils 201 sind auf der Unterseite der rechteckigen Aussparung 415 beidseitig der Austrittsöffnung 323 umlaufend Dichtrippen 321 angeordnet, welche an ihrer Oberseite jeweils eine Auflagefläche 321 und somit einen Dichtsitz zu der Unterseite des Elastomer-Bandes 205 aufweisen. Ansonsten ist das Überdruckventil 201 wie oben zur Figur 6 beschrieben ausgebildet. Da das Elastomer-Band 205 mit seinen Seiten an den Seitenwänden der rechteckigen Aussparung 415 anliegt, an seiner Unterseite jedoch nur auf den Auflageflächen 321 der Dichtrippen 307 aufliegt, hebt das Elastomer-Band 205 im Falle eines kritischen Überdruckes und somit einer durch die Entlastungsbohrung 203 strömenden Druckluft schnell von den Auflageflächen 231 ab und aufgrund der beidseitigen freihängenden Ränder des Elastomer-Bandes 205 wird dieses bevorzugt in diesen Randbereichen weiter verstärkt abgehoben, durch welche die Druckluft aus der rechteckigen Aussparung 415 in die Umgebung 309 austritt.

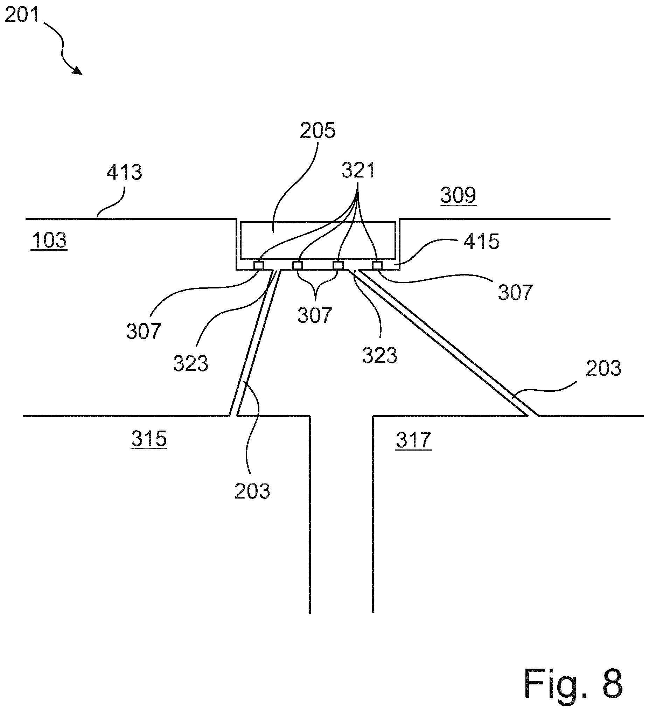

In einer weiteren Alternative des Überdruckventils 201 weist die Trägereinheit 103 einen ersten Innenraum 315 und einen zweiten Innenraum 317 auf (Figur 8). Die beiden Innenräume 315 und 317 sind jeweils mit einer separaten Entlastungsbohrung 203 mit einer einzigen rechteckigen Aussparung 415 verbunden. Die beiden Austrittsöffnungen 323 der beiden Entlastungsbohrungen 203 sind jeweils von Dichtrippen 307 innerhalb der rechteckigen Aussparung 415 umgeben, wodurch zwischen den beiden Austrittsöffnungen 323 mittig zwei Dichtrippen 307 angeordnet sind und eine redundante Abdichtung zwischen den beiden Entlastungsbohrungen 203 gewährleisten. Die Oberseiten der Dichtrippen 307 bilden, wie oben beschrieben, die Auflageflächen 321 und somit einen Dichtsitz für das Elastomer-Band 205 aus. Durch die umlaufenden Dichtrippen 307 in der rechteckigen Aussparung 415 liegen umlaufende Dichtlinien jeweils um die Austrittsöffnung 323 vor, dadurch wird ein Überströmen von Druckluft von dem ersten Innenraum 315 über die jeweilige Entlastungsbohrung 203 in den zweiten Innenraum 317 oder umgekehrt verhindert. Ansonsten ist die in Figur 8 gezeigte Alternative des Überdruckventils 201 wie oben beschrieben aufgebaut.

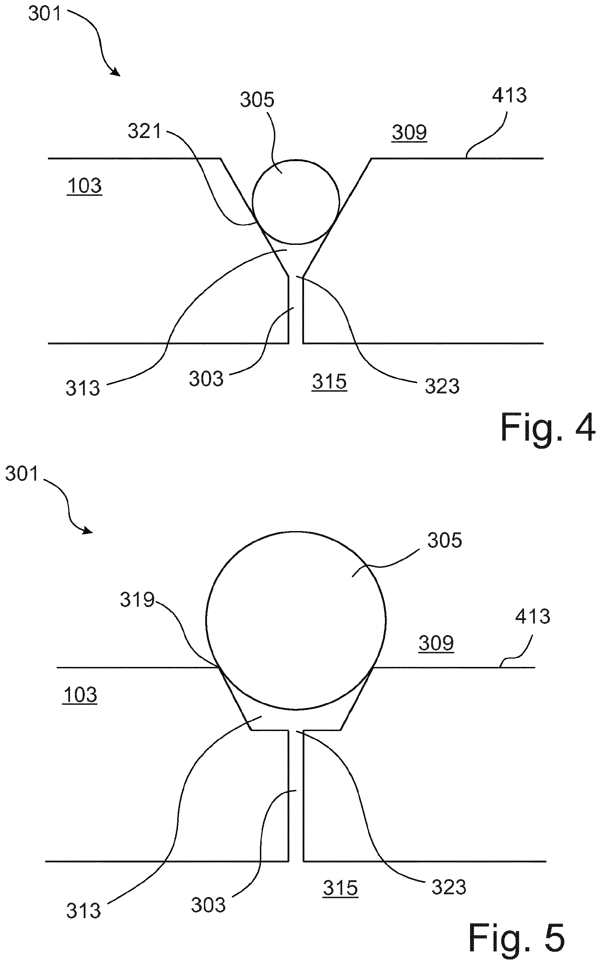

In einer weiteren in Figur 4 gezeigten Alternative des Überdruckventils weist ein Überdruckventil 301 eine konische Aussparung 313 auf, welche sich von der Außenwand 413 der Trägereinheit 103 zu der Austrittsöffnung 323 konisch verengt und anschließend in einen Entlastungskanal 303 übergeht, welcher an seinem anderen Ende mit einem Innenraum 315 der Lithotripsievorrichtung 101 verbunden ist. In die konische Aussparung 313 ist ein Dichtring 305 eingepresst worden, welcher an einer Auflagefläche 321 der Seitenwände der konischen Aussparung 313 eingeklemmt ist. Im Falle eines Auftretens eines kritischen Überdruckes strömt die Druckluft von dem Innenraum 315 durch den Entlastungskanal 303 und die Austrittsöffnung 323 in den unteren engeren Bereich der konischen Aussparung 313, wodurch der Dichtring 305 sich von der Auflagefläche 321 löst und sich beschleunigt aufgrund der konischen Erweiterung in Richtung zur Umgebung 309 abhebt.

In einer weiteren in Figur 5 gezeigten Alternative des Überdruckventils 301 ist ein Dichtring 305 nicht vollständig in einer konischen Aussparung 313 aufgenommen, sondern weist eine deutlich höhere Höhe als die konische Aussparung 313 auf, wodurch der Dichtring 305 größtenteils in die Umgebung 309 ragt. Durch die Ausmaße des Dichtrings 305 liegt dieser an seiner Unterseite nicht auf der Unterseite der konischen Aussparung 313 um die Austrittsöffnung 323 auf, sondern bildet am Rande der konischen Aussparung 313 zu der Außenwand 413 eine rundumlaufende Dichtlinie 319. Dadurch ist das Überdruckventil 301 aufgrund des Dichtringes 305 überwiegend gewichtsbelastet ausgelegt und im Falle eines kritischen Überdruckes muss die durch den Entlastungskanal 303 und die Austrittsöffnung 323 in die konische Aussparung 313 eintretende Druckluft entsprechend vornehmlich das Gewicht des Dichtungsringes 305 überwinden, um den Dichtungsring 305 an der Dichtlinie 319 von der konischen Aussparung 313 zu lösen.

Somit werden in der Lithotripsievorrichtung 101 Überdruckventile 201, 301 bereitgestellt, mit denen sicher und zuverlässig Innenräume 315 entlüftet werden können, welche ungewollt mit Druckluft beaufschlagbar sind, bevor die Druckluft mit einem kritischen Überdruck über die Hohlsonde 311 in den Körper eines Patienten gelangen kann.

Die Erfindung betrifft ein medizinisches Instrument zum Behandeln eines Körpers, wobei das medizinische Instrument eine Trägereinheit mit einer Außenseite zu einer Umgebung und mindestens einen ersten Innenraum innerhalb der Trägereinheit aufweist, und der Innenraum mit einem Überdruck beaufschlagbar ist, wobei das medizinische Instrument eine Druckentlastungseinrichtung mit mindestens einem elastischen Dichtelement aufweist, wobei das mindestens eine elastische Dichtelement in einer ersten Aussparung in und/oder an der Außenseite der Trägereinheit angeordnet ist und der mindestens eine Innenraum mittels zumindest einem Druckentlastungskanal mit der ersten Aussparung verbunden ist, sodass bei einem Beaufschlagen des mindestens einen Innenraums mit dem Überdruck der Überdruck mittels eines Freigebens der ersten Aussparung durch das mindestens eine elastische Dichtelement in die Umgebung abgebbar ist. Die Zeichnungen, die Beschreibung und die Ansprüche enthalten zahlreiche Merkmale in Kombination. Es versteht sich, dass die vorstehend genannten Merkmale nicht nur in der jeweils angegebenen Kombination, sondern auch in anderen Kombinationen oder in Alleinstellung verwendbar sind, ohne den Rahmen der vorliegenden Erfindung zu verlassen.

### Bezugszeichenliste

- 101: Lithotripsievorrichtung
- 103: Trägereinheit
- 105: Gehäuserohr
- 107: Proximale Endkappe
- 109: Proximale Gegenmutter
- 111: Distale Endkappe
- 113: Distale Gegenmutter
- 115: Distale Richtung
- 117: Bedienelement
- 119: Absaugleitung
- 121: Führungsrohr
- 123: Erste Durchgangsbohrung für Abluft
- 124: Zweite Durchgangsbohrung für Abluft
- 125: Dritte Durchgangsbohrung für Abluft
- 126: Vierte Durchgangsbohrung für Abluft
- 127: Fünfte Durchgangsbohrung für Zuluft
- 131: Steuerhülse
- 133: Erste Ventilbohrung
- 134: Zweite Ventilbohrung
- 135: Dritte Ventilbohrung
- 136: Vierte Ventilbohrung
- 141: Hohlraum/Beschleunigungsstrecke
- 143: Projektil
- 145: Mitnehmerring
- 149: Längsmittelachse
- 151: erster Abluftanschluss
- 153: zweiter Abluftanschluss
- 155: erster Zuluftanschluss
- 156: zweiter Zuluftanschluss
- 157: Zuluftkammer
- 159: Abluftkammer
- 171: Rückholfeder
- 173: Halterung
- 181: Tempierfeder
- 183: Halter
- 185: Hüllrohr
- 187: Proximale Abschlusskappe
- 189: Distale Abschlusskappe
- 191: Polymer-Scheiben
- 193: O-Ring
- 195: Schweißring
- 201: Überdruckventil
- 203: Entlastungsbohrung
- 205: Elastomer-Band
- 211: Sonotrode
- 213: Sonotrodenspitze
- 215: Kopfstück
- 216: Führungsteil
- 217: O-Ring
- 219: Bremselement
- 221: Querbohrung
- 223: Stößel
- 225: Betätigungsgriff
- 301: Überdruckventil
- 303: Entlastungskanal
- 305: Dichtring
- 307: Dichtrippe
- 309: Umgebung
- 311: Hohlsonde
- 313: konische Aussparung
- 315: Innenraum
- 317: zweiter Innenraum
- 319: Dichtlinie
- 321: Auflagefläche
- 323: Austrittsöffnung
- 413: Außenwand
- 415: rechteckige Aussparung

## Patentansprüche

1. Medizinisches Instrument (101) zum Behandeln eines Körpers, wobei das medizinische Instrument (101) eine Trägereinheit (103) mit einer Außenseite (413) zu einer Umgebung (309) und mindestens einen ersten Innenraum (315, 317) innerhalb der Trägereinheit (103) aufweist, und der Innenraum (315, 317) mit einem Überdruck beaufschlagbar ist, **dadurch gekennzeichnet, dass** das medizinische Instrument (101) eine Druckentlastungseinrichtung (201, 301) mit mindestens einem elastischen Dichtelement (205, 305) aufweist, wobei das mindestens eine elastische Dichtelement (205, 305) in einer ersten Aussparung (313, 415), in und/oder an der Außenseite (413) der Trägereinheit (103) angeordnet ist und der mindestens eine Innenraum (315, 317) mittels zumindest einem Druckentlastungskanal (203, 303) mit der ersten Aussparung (313, 415) verbunden ist, sodass bei einem Beaufschlagen des mindestens einen Innenraums (315, 317) mit dem Überdruck der Überdruck mittels eines Freigebens der ersten Aussparung (313, 415) durch das mindestens eine elastische Dichtelement (205, 305) in die Umgebung (309) abgebbar ist.

2. Medizinisches Instrument (101) nach Anspruch 1, **dadurch gekennzeichnet, dass** die Druckentlastungseinrichtung (201, 301) ein zweites elastisches Dichtelement in einer zweiten Aussparung in und/oder an der Außenseite der Trägereinheit, ein drittes elastisches Dichtelement in einer dritten Aussparung in und/oder an der Außenseite der Trägereinheit und/oder ein weiteres elastisches Dichtelement (205, 305) in einer weiteren Aussparung (313, 415) in und/oder an der Außenseite (413) der Trägereinheit (103) aufweist.

3. Medizinisches Instrument (101) nach Anspruch 1 oder 2, **dadurch gekennzeichnet, dass** in der Trägereinheit (103) ein zweiter Innenraum (317), ein dritter Innenraum, ein vierter Innenraum und/oder weitere Innenräume angeordnet ist oder sind, wobei der jeweilige Innenraum (315, 317) mit einem Überdruck beaufschlagbar und über jeweils einen Druckentlastungskanal (203, 303) mit der ersten Aussparung, der zweiten Aussparung, der dritten Aussparung und/oder mit der weiteren Aussparung (313, 415) verbunden ist.

4. Medizinisches Instrument (101) nach einem der vorherigen Ansprüche, **dadurch gekennzeichnet, dass** die erste Aussparung, die zweite Aussparung, die dritte Aussparung und/oder die weitere Aussparung (313, 415) jeweils mit zwei oder mehreren Druckentlastungskanälen (203, 303) verbunden ist oder sind.

5. Medizinisches Instrument (101) nach einem der vorherigen Ansprüche, **dadurch gekennzeichnet, dass** das elastische Dichtelement, das jeweilige elastische Dichtelement oder die elastischen Dichtelemente (205, 305) als elastisches Dichtband (205) und/oder elastischer Dichtring (305) ausgebildet ist oder sind.

6. Medizinisches Instrument (101) nach einem der vorherigen Ansprüche, **dadurch gekennzeichnet, dass** die Aussparung, die jeweilige Aussparung oder die Aussparungen (313) an ihrer Seitenwand oder ihren Seitenwänden konisch geformt ist oder sind.

7. Medizinisches Instrument (101) nach einem der vorherigen Ansprüche, **dadurch gekennzeichnet, dass** die Aussparung, die jeweilige Aussparung oder die Aussparungen (313, 415) eine geformte Auflagefläche (321) für das jeweilige elastische Dichtelement (205, 305) aufweist oder aufweisen.

8. Medizinisches Instrument (101) nach einem der vorherigen Ansprüche, **dadurch gekennzeichnet, dass** die Aussparung, die jeweilige Aussparung oder die Aussparungen (313, 415) eine Dichtrippe (307) oder zwei oder mehrere Dichtrippen (307) aufweist oder aufweisen.

9. Medizinisches Instrument (101) nach einem der vorherigen Ansprüche, **dadurch gekennzeichnet, dass** das medizinische Instrument (101) eine Fluiddurchführung (221) zum Abtransportieren eines zertrümmerten Steinfragmentes und/oder zum Wärmetransport aufweist.

10. Medizinisches Instrument (101) nach einem der vorherigen Ansprüche, **dadurch gekennzeichnet, dass** das medizinische Instrument eine ballistische Lithotripsievorrichtung (101), insbesondere eine ballistische intrakorporale Lithotripsievorrichtung, zum Zertrümmern von Körpersteinen ist, wobei die Lithotripsievorrichtung (101) ein Führungsrohr (121) mit einem Hohlraum (141), mit einem proximalen Ende und mit einem distalen Ende, ein bewegbares Projektil (143), ein distalseitiges Anschlagselement und ein proximalseitiges Anschlagselement für das bewegbare Projektil (143) aufweist, wobei das Führungsrohr (121) zumindest teilweise in der Trägereinheit (103) angeordnet ist, und der Lithotripsievorrichtung (101) eine Antriebseinrichtung zum Hin- und Herwegen des Projektils (143) zwischen dem distalseitigen Anschlagselement und dem proximalseitigen Anschlagselement und eine Sonotrode (211) zuordenbar sind, und die Sonotrode (211) an ihrem proximalen Ende mit der Trägereinheit (103) und/oder dem Führungsrohr (121) direkt oder indirekt verbindbar und durch ein mechanisches Auftreffen des Projektils (143) auf das distalseitige Anschlagselement schwingungsanregbar ist, wobei in einem Fehlerfall der jeweilige Innenraum (315, 317, 185, 215) mit dem Überdruck aufgrund einer Kompression in dem Hohlraum (141) des Führungsrohres (121) durch das Hin- und Herbewegen des Projektils (143) beaufschlagbar ist.

11. Medizinisches Instrument (101) nach einem der vorherigen Ansprüche, **dadurch gekennzeichnet, dass** das medizinische Instrument eine pneumatische Lithotripsievorrichtung (101) ist, und mittels der Antriebseinrichtung ein Druckmedium in ein Inneres der Trägereinheit (103) und/oder des Führungsrohres (121) zum Hin- und Herbewegen des Projektils (143) in den Hohlraum (141) des Führungsrohres (121) zu- und/oder abführbar ist, wobei in einem Fehlerfall mittels des Druckmediums der jeweilige Innenraum (315, 317, 185, 215) mit einem Überdruck beaufschlagbar ist.

12. Medizinisches Instrument (101) nach einem der vorherigen Ansprüche, **dadurch gekennzeichnet, dass** die Sonotrode (211) eine Hohlsonde (311) ist.
